# EUROPEAN PATENT APPLICATION

(11) **EP 3 569 195 A1**
(43) Date of publication of application: **20.11.2019**
(21) Application number: 18386013.9
(22) Date of filing: 16.05.2018
(51) Int. Cl.: A61F 2/24

(54) **SELECTIVELY ADJUSTABLE ANNULOPLASTY RING**

(71) Applicant: Vasdeki, Efthalia, 145 69 Anoixi Attkis (GR); Filandrianos, Georgios, 115 24 Nea Filothei Attkis (GR); Cheilas, Vasileios, 157 73 Zografou Attkis (GR)
(72) Inventor: Vasdeki, Efthalia, 145 69 Anoixi Attkis (GR); Filandrianos, Georgios, 115 24 Nea Filothei Attkis (GR); Cheilas, Vasileios, 157 73 Zografou Attkis (GR)

(57) **Abstract**

A Selectively Adjustable annuloplasty ring for correction of the valve insufficiency of a human heart includes a sewing cuff 17 , a plurality of basic functional units 18-27 ,each one consisting of a nitinol spring 2,a catheter's socket 4 and a heating wire 3 ,that have the ability to shrink autonomously in size when heat is applied .The heat transferred to the nitinol spring 2 is a result of the heating wire's 3 temperature raise when a catheter is being connected to the catheter's socket magnetic base 8,9 and starts transferring power to the basic functional unit selected .Thus ,the annuloplasty ring has the ability to shrink partially in size depending on the basic functional unit activated so that the heart valve is corrected in a more efficient way ,thereby correcting blood regurgitation improving the patient's life.

## Description

The invention is generally related with devices and techniques intended to repair the cardiac valves and specifically an annuloplasty device of that shape that resembles the normal fibrous ring of any of the cardiac valves (aortic, pulmonary, mitral, tricuspid) and restores the shape of an enlarged or insufficient fibrous ring of any of the cardiac valves, to the shape that is closer to the normal.

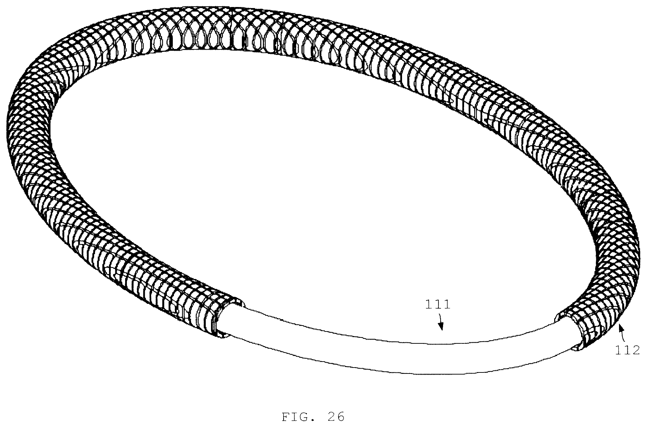

In the normal human heart there are four valves a)the tricuspid valve , b)the mitral valve ,c)the aortic valve and d)the pulmonary valve .The tricuspid valve controls the flow between the right atrium and the right ventricle , the pulmonary valve regulates the blood flow between the right ventricle and the lungs ,the mitral valve controls the blood flow between the left atrium and the left ventricle whereas the aortic valve regulates the flow from the left ventricle to the aorta. However, sometimes the cardiac valves tend to function abnormally and lead to heart valve disease. To be more specific the fibrous ring around the cardiac valve can change it's normal size and grow in diameter. In the mitral valve the causes are usually cardiomyopathy, endocarditis, rheumatic heart disease or mitral valve prolapse whereas the tricuspid valves usually affected from conditions such as pulmonary hypertension and congestive heart failure. The stretch of the fibrous valvular ring causes it's diameter increasement and leads to abnormal closure between the valvular flaps, which causes blood regurgitation. This condition is generally treated by surgical restoration of the valve (mitral-clip) or with an annuloplasty ring. The artificial annuloplasty ring is sewed around the fibrous valvular ring and is capable of following the dynamic changes that happen to the heart and the fibrous ring during systole and diastole. Furthermore the dimensions of the valve's annulus are following a dynamic process which is subjected to the hemodynamic and volumetric status of the heart. Annuloplasty rings are capable of maintaining the valve leaflets and the valvular ring in their normal position avoiding blood regurgitation from happening, thus allowing normal blood flow. It's worth mentioning that there are different kinds of annuloplasty rings that tend to differ in size, shape even in mechanical properties. The most common shape, is the D-shape that resembles the letter D of the Latin alphabet and can be closed or partially open (C- shape). Furthermore, artificial annuloplasty rings can be rigid, semi-rigid or elastic, each one of which follows a different valve kinematics . Usually, a conventional annuloplasty ring as shown in Fig 26 is made of semi rigid materials, having a super elastic alloy core 111 with carbon film coating 112 whereas titanium alloy and Elgiloy alloy are also some of the construction materials. Implanting, those annuloplasty rings requires open heart surgery while transdermal catheterization is a recent option, still in experimental phase. It's worth mentioning, that the size of the annuloplasty ring is pre-determined and changing after implantation is not an option. Depending on the valve that needs to be repaired ,an annuloplasty ring of certain shape and size is being selected and sewed on the abnormal's valve fibrous ring, correcting it's form, thus preventing blood regurgitation. A related to point to consider is that there are annuloplasty rings whose size can change mechanically only during surgery, with the traction of small ropes.

The objective of the present invention is to provide an annuloplasty ring and more particular an annuloplasty ring for the heart whose size can selectively change after implantation without an open heart surgery procedure.

In accordance with the present invention, there is provided an annuloplasty ring ,comprising a plurality of basic functional units, non-conductively connected to each other, each one consisting of a nitinol spring ,a catheter's socket and a heating wire which is connected to the magnetic base of the catheter's socket ,all being surrounded by a sewing cuff formed from the combination of an inner layer of heat insulating material and an outer layer made of knitted double velour polyester (Dacron).

Advantageously, the annuloplasty ring has the ability to shrink partially in size in vivo ,depending on the basic functional unit activated, so that the heart valve is corrected in a more efficient way ,thereby correcting blood regurgitation improving the patient's life.

Further benefits and advantages of the present invention will become apparent after a careful reading of the detailed description with appropriate reference to the accompanying drawings.

In the drawings:
Fig 1: Is a perspective view of the basic functional unit of the annuloplasty ring.
Fig 2: Is a plan view of the basic functional unit of the annuloplasty ring as shown in Figure 1.
Fig 3: Is an exploded view of the annuloplasty's ring catheter's socket.
Fig 4:Is an exploded view of the annuloplasty ring.
Fig 5: Is a perspective view of the annuloplasty ring without the sewing cuff in it's before implantation position.
Fig 6: Is a perspective view of the annuloplasty ring without the sewing cuff, where the circumference is reduced after the adjustment of some of the basic functional units .
Fig 7: Is a perspective view of the annuloplasty ring without the sewing cuff, where the circumference is reduced after the adjustment of some of the basic functional units .
Fig 8: Is a perspective view of the annuloplasty ring without the sewing cuff where the circumference is reduced after the adjustment of some of the basic functional units .
Fig 9: Is a perspective view of a D-shape annuloplasty ring without the sewing cuff.
Fig 10: Is a perspective view of an other version of the annuloplasty ring without the sewing cuff, which is capable of increasing it's circumference.
Fig 11: Is a perspective view of an other version of the annuloplasty ring without the sewing cuff, which is capable of increasing it's circumference after some of the basic functional units are activated.
Fig 12: Is a perspective view of the annuloplasty ring in it's initial position including the sewing cuff.
Fig 13: Top sectional view of a normal heart at the plane of the four valves.
Fig 14: Top sectional view of the heart at the plane of the four valves where there is an insufficiency of the mitral valve.
Fig 15: Top sectional view of the heart at the plane of the four valves, as shown in Figure 14, where the insufficiency of the mitral valve is corrected by an annuloplasty ring.
Fig 16: Top sectional view of the heart at the plane of the four valves, as shown in Figure 15, where there is an other kind of insufficiency of the mitral valve after the initial correction - implantation.
Fig 17: Top sectional view of the heart at the plane of the four valves, as shown in Figure 16, where the new insufficiency of the mitral valve is corrected after the annuloplasty's ring adjustment in vivo.
Fig 18: Top sectional view of the heart at the plane of the four valves where there is an insufficiency of the tricuspid valve.
Fig 19: Top sectional view of the heart at the plane of the four valves, as shown in Figure 18, where the insufficiency of the tricuspid valve is corrected by a D-shape annuloplasty ring.
Fig 20: Top sectional view of the heart at the plane of the four valves, as shown in Figure 19, where there is an other kind of insufficiency of the tricuspid valve after the initial correction - implantation.
Fig 21: Top sectional view of the heart at the plane of the four valves, as shown in Figure 20, where the new insufficiency of the tricuspid valve is corrected after the annuloplasty's ring adjustment in vivo.
Fig 22: Cross sectional view of the heart where there is an other kind of insufficiency of the tricuspid valve after the initial correction - implantation as shown in Fig 20.
Fig 23: Cross sectional view of the heart where the new insufficiency of the tricuspid valve is corrected after the annuloplasty's ring adjustment in vivo as shown in Fig 21.
Fig 24: Cross sectional view of the heart where there is an other kind of insufficiency of the mitral valve after the initial correction - implantation as shown in Fig 16.
Fig 25: Cross sectional view of the heart where the new insufficiency of the mitral valve is corrected after the annuloplasty's ring adjustment in vivo as shown in Fig 17.
Fig 26: Is a perspective view of a conventional annuloplasty ring for the human heart in accordance with the prior art.

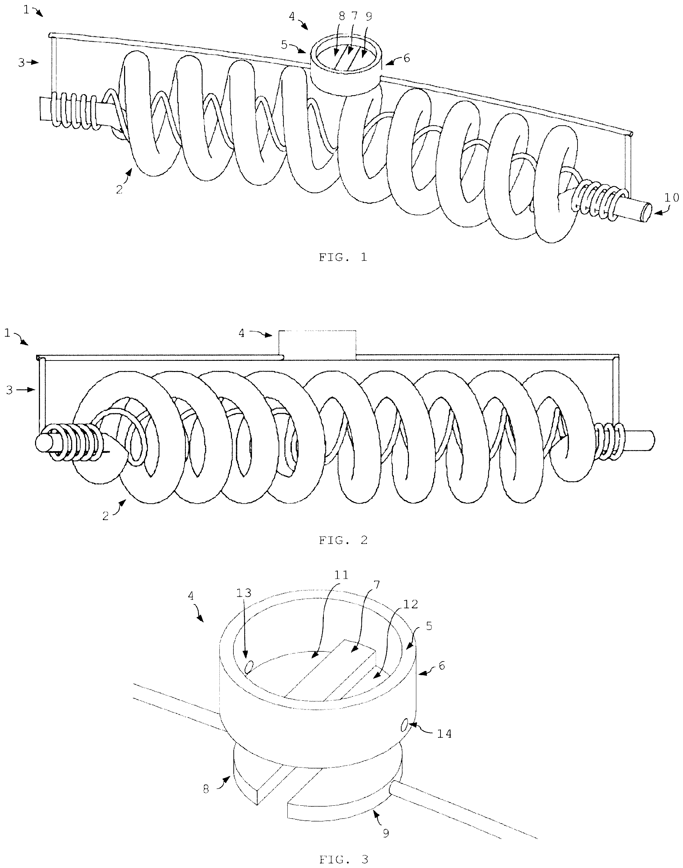

Referring to the drawings and initially to Figs 1-12 , an annuloplasty ring for a human heart in accordance with the preferred embodiment of the present invention comprises a plurality of basic functional units 1, non-conductively connected to each other, each one consisting of a nitinol spring 2 ,a catheter's socket 4 and a heating wire 3 which is connected to the magnetic bodies 8,9 of the catheter's socket 4 ,all being surrounded by a sewing cuff 17 formed from the combination of an inner layer 73 of heat insulating material and an outer layer 72 made of knitted double velour polyester (Dacron).

Each nitinol spring 2 is connected to each other with non-conductive material 10 thus avoiding heat transfer between basic functional units during the ring's circumference adjustment. In this point is worth mentioning that if the nitinol springs were conductively connected, heat would be transmitted to the adjacent basic functional units leading to their shrinkage. The same thing would happen if instead of a plurality of non-conductively connected smaller springs there was one bigger spring.

The heating wire 3 is mounted on the inside of each nitinol spring 2 and has the shape of a smaller coil as shown in Figs 1,2 in order to be able to follow the basic functional unit's kinematics during activation. It's worth mentioning that after being coiled to the ends of each nitinol spring 2 , the heating wire 3 passes through the sewing cuff 17 and the insulating part of the catheter's socket 5 and is conductively connected to the magnetic bodies 8,9 as shown in Fig 3.

Nitinol is a nickel-titanium alloy with the typical ratio 40-50% nickel, 50-60% titanium known as a shape memory alloy, a metal alloy that demonstrates pseudo-elasticity and the shape memory effect. The shape memory effect is when a material returns to its pre-deformed shape by heating. The initial position of the nitinol springs is when the coils are closed, which means that the spirals of each spring are in touch with each other. However, before the annuloplasty ring is placed into the heart, the springs are open which means that the spirals of each coil are not in touch with each other, meaning that each spring is stretched .Due to nitinol's property to return to it's initial form when heated, it is easily understood that when a coil is heated it will start shrinking until is fully closed meaning that each spiral will be in touch with each other, thus the annuloplasty ring comprising of these basic functional units has the ability to partially decrease it's circumference.

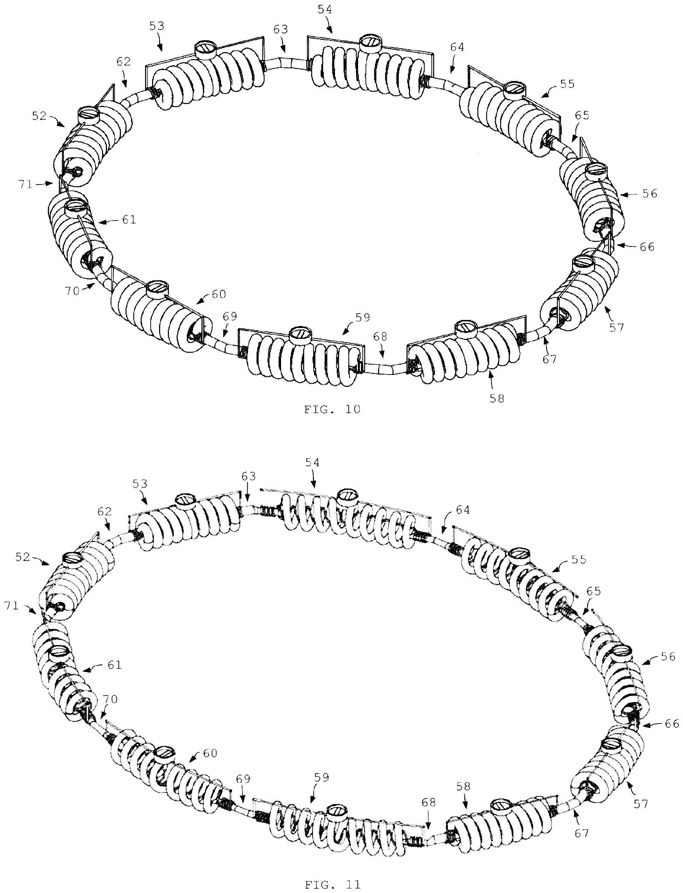

In this point is worth mentioning that the initial position of each nitinol spring could be the exact opposite as the one described above meaning that the spirals of each coil are not in touch with each other, thus each spring is stretched. Before implantation though, each spring is closed meaning that the spirals of each spring are in touch with each other. It is obvious that when heated, each spring will start increasing it's length meaning that the annuloplasty ring comprising of these basic functional units has the ability to partially increase it's circumference .This is better shown in Fig 10 where the springs of the basic functional units 52-61 ,which are non-conductively connected to each other with the insulation materials 62-71, are fully closed before implantation. After activation of the basic functional units 54,55,56,59,60,61 as shown in Fig 11 though, each one of these springs returns to it's initial position meaning that their length is increased.

Unless otherwise specified, from now on when we refer to the annuloplasty ring or it's compartments, the initial position of the nitinol springs is set to the one where the coils are closed, which means that the spirals of each spring are in touch with each other. However, before the annuloplasty ring is placed into the heart, the springs are open which means that the spirals of each coil are not in touch with each other, meaning that each spring is stretched .Due to nitinol's property to return to its initial form when heated, it is easily understood that when a coil is heated it will start shrinking until is fully closed meaning that each spiral will be in touch with each other, thus the annuloplasty ring comprising of these basic functional units has the ability to partially decrease it's circumference.

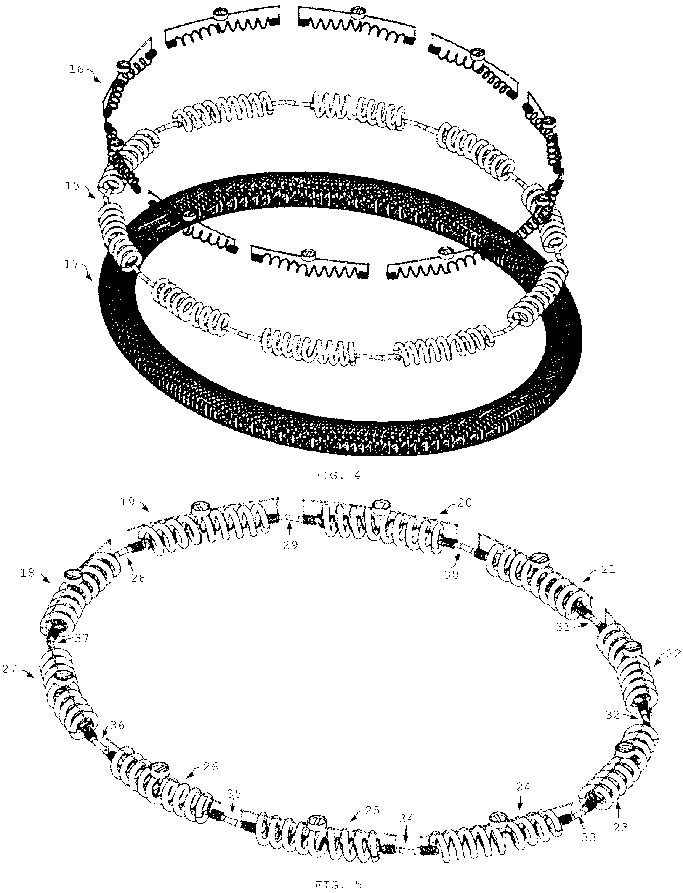

At the inside of the nitinol springs in series assembly 15 there is a number of heating wires 16 as shown in Fig 4 ,same as the one of the nitinol coils, since each heating wire is being located at the inside of each nitinol spring with the form of a smaller coil. The heating wires are made of materials like Kanthal, Nichrome, Constantan, Balco etc. which have the property to change their temperature really fast without energy lost.

The catheter's socket 4 consists of three kind of different materials and is better shown in Fig 3. The majority of the socket is made out of insulating material 5 whereas at the inside of it there are two magnetic bodies 8,9 each one of which is conductively connected with the heating wire 3 . The insulating part of the catheter's socket 5 is made out of non-conductive material and has the shape of a hollow cylinder whose bottom is closed, and has a specific formation with an extrusion 7 being the main characteristic of it .The non-conductive extrusion creates two sockets 11, 12 for the placement of the two magnetic bodies 8,9 and acts as an insulator thus preventing the two magnetic bodies from being conductively connected to each other when current is applied. Furthermore, the outer layer 6 of the catheter's socket is made of radiopaque material making the distinction between the basic functional units easier during catheterization. Additionally it is obvious that each catheter's socket corresponds to a different basic functional unit ,thus, depending on the section of the valvular annulus that needs to be corrected , a different basic functional unit is activated. In this point is worth mentioning that the term 'basic functional unit activation' refers to the transportation of current from the catheter to the heating wire which is heated and causes the nitinol's spring temperature to rise thus making it return to it's initial position.

The magnetic bodies 8, 9 have the shape of a semicylinder and after being placed to their sockets 11,12 are conductively connected to the heating wire 3 which passes through the catheter's socket insulating part 5 from the holes 13,14 as shown in Fig 3.

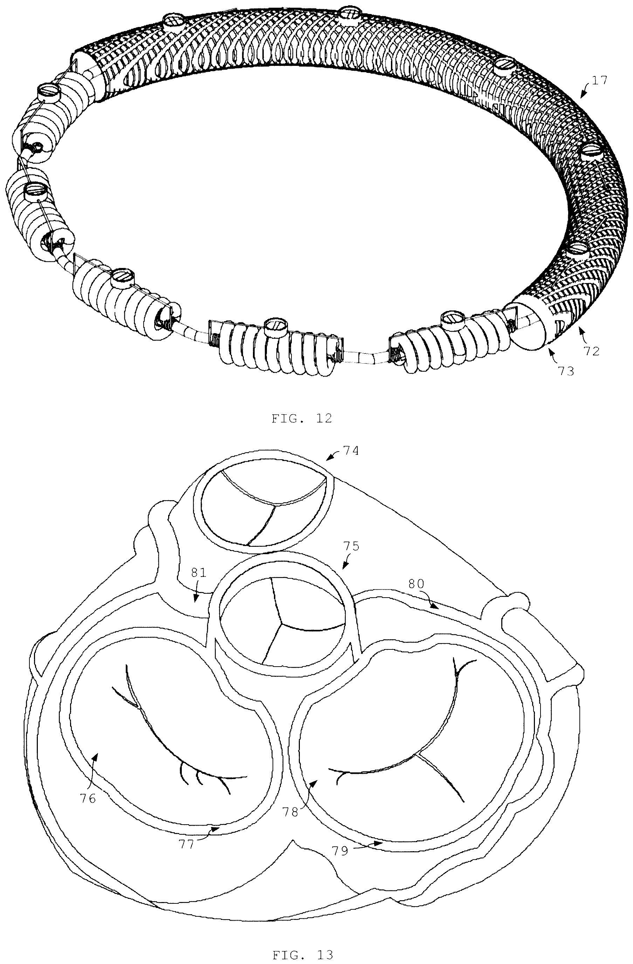

The sewing cuff 17 that surrounds the basic functional units consists of two different layers as shown in Fig 12 .The inner layer 73 is made out of non-conductive and heat insulating, elastic material that prevents leaking of heat and current during activation of any of the basic functional units. Then, the outer layer 72 of the sewing cuff is made out of knitted double velour polyester that during the ring's circumference adjustment can follow the nitinol spring's reduction without causing local stenosis.

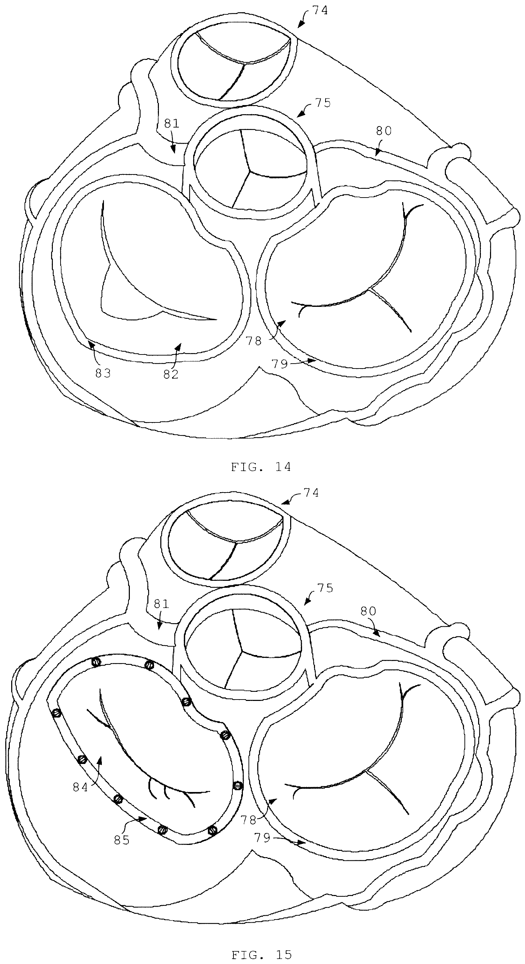

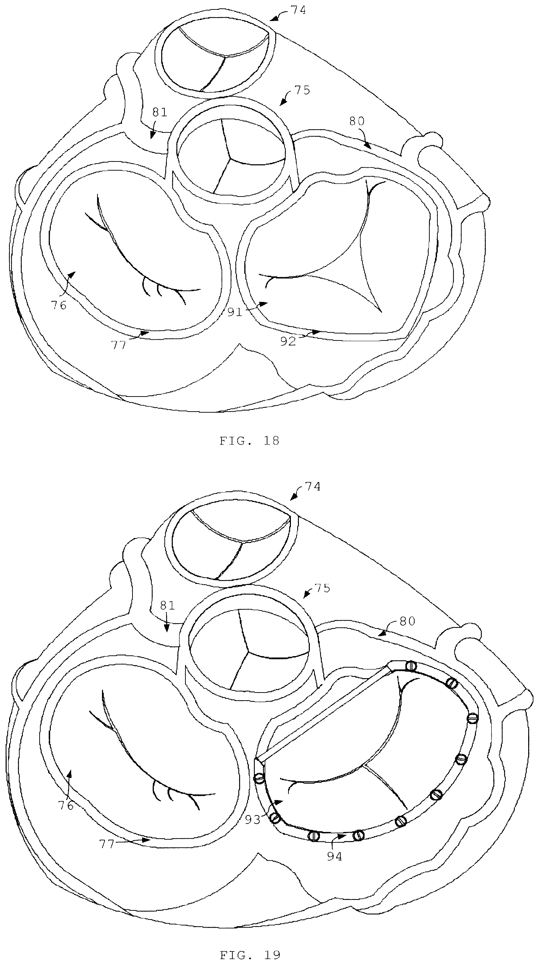

As mentioned before annuloplasty rings find application in both the mitral and the tricuspid valve. A plane section of the heart's valves as shown in Fig 13 make it easier to understand the normal anatomy and the surrounding formations. As previously stated, the human heart has four valves: the pulmonary 74, the aortic 75, the mitral 76 with it's fibrous ring 77 and the tricuspid 78 with it's fibrous ring 79. In addition, just for explanatory reasons, the main arteries of the heart, the right coronary with its branches 80 and the left coronary artery 81 with its branches are also shown in Figs 13-21. In these figures someone can also notice the two flaps of the mitral valve 76 and the three flaps of the tricuspid valve 78. As said before the annuloplasty ring described finds application on both valves. Now, let's assume that the tricuspid valve 91 due to its ring 92 tension, is insufficient, causing blood to regurgitate during systole as shown in Fig 18 while the other structures remain normal. The same thing can happen to the mitral valve 82 and its ring 83 as shown in Fig 14, with the same mechanism, causing a small blood flow to the opposed direction (from left ventricle to the left atrium) meaning that when the next diastole circle begins the ventricle will suffer a volume load and over time this will lead to heart failure.

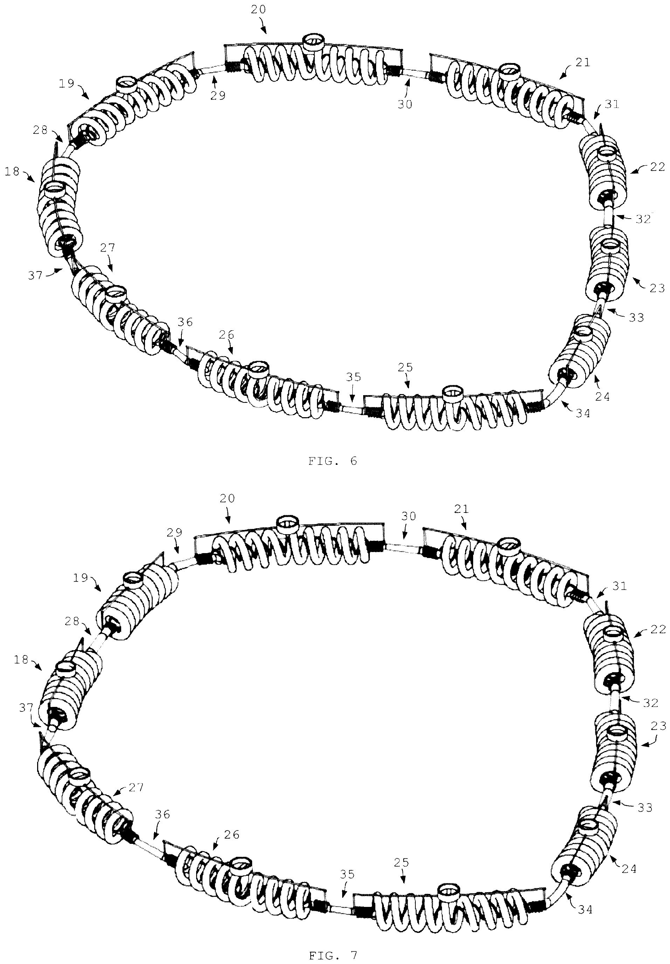

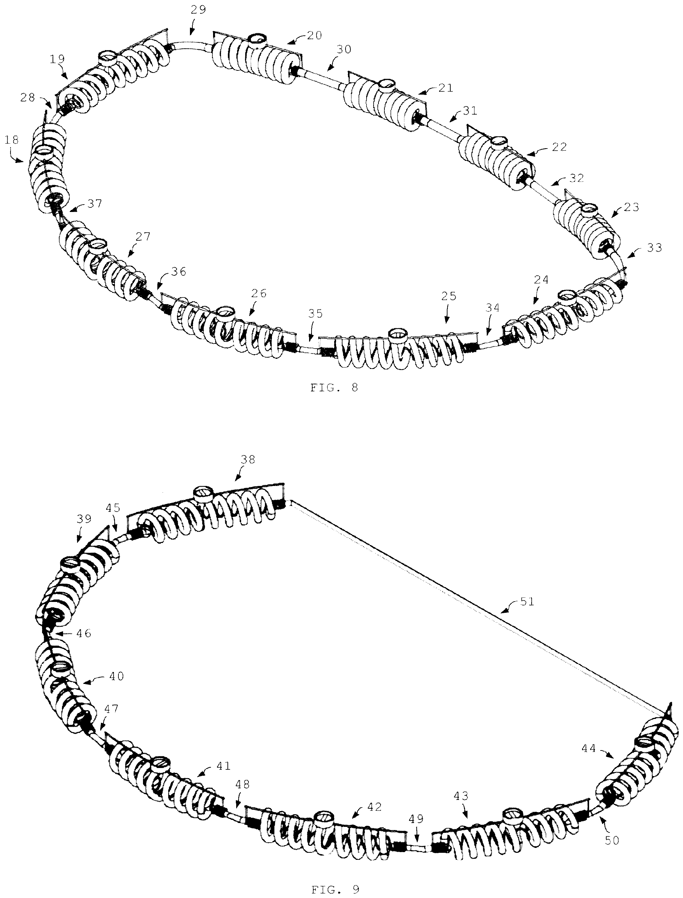

Annuloplasty rings are the best treatment option for these conditions, but sometimes, due to predetermined size and total circumferential reduction of the valve's fibrous ring diameter, while treating the blood regurgitation might cause a local valve stenosis. So it is obvious that correction of the valve insufficiency is a procedure which is highly personalized depending on the structural disorder (size, shape) of a person's mitral or tricuspid valve. So it's easy for someone to understand that restoration of a heart valve's structure must be done in such manner that no stenosis or insufficiency remains. That can be done by correcting the fibrous ring size not totally but partially as shown in Fig 6 where the basic functional units 22,23,24 are closed after activation while the basic functional units 18,19,20,21,25,26,27 remain open meaning that the fibrous ring that the annuloplasty ring is sewed on, will shrink in diameter the same way as ring. In Fig 7 there is an other version of the annuloplasty's ring selective circumference reduction where the basic functional units 20,21,25,26,27 remain open whereas the basic functional units 18,19,22,23,24 are closed after activation .The same thing applies to the Fig 8 where the basic functional units 18,19,24,25,26,27 remain open whereas the basic functional units 20,21,22,23 are closed after activation .As it is obvious from the Figs 5-8 the annuloplasty ring described in this patent , depending on the basic functional unit activated , is capable of correcting selectively and specifically any kind of valvular insufficiency.

In this point it's worth mentioning that restoration of both the mitral and the tricuspid valve is different due to the surrounding anatomy of each valve. As indicated previously an annuloplasty ring can be closed or partially open (C-shape). Both of these formations are capable of correcting the regurgitation of both the mitral and the tricuspid valve. The main advantage of the closed shape is the stability that offers to the valve's annulus whereas the open shape is generally preferred because of the lack of pressure that is being applied to the surrounding formations of each valve. The most important formation that a circular prosthesis (closed shape ring) would affect around the tricuspid valve would be the heart's conduction system whereas a circular prosthesis sewed on the mitral valve would be too close to the aortic valve and could lead to mitral and left ventricular dysfunction.

As earlier explained, correction of the valve's regurgitation, is usually the result of the valve's annulus diameter reduction. An annuloplasty ring of specific size is being selected and sewed on the heart valve's annulus correcting the insufficiency by reducing its size. However annuloplasty rings that have the ability to shrink in size after placement may offer a better solution depending on each valve's unique characteristics. As anyone can understand the reduction of the annuloplasty's ring diameter is the result of it's perimeter's reduction meaning that the insufficiency correction is also the result of the annuloplasty's ring perimeter reduction. In this point is worth mentioning that only a closed shape can reduce it's diameter by reducing its perimeter. The annuloplasty ring described in this patent is based in this principle meaning that it can't have the formation of an open shape. However the alternative formation that is shown in Fig. 9 solves the problem mentioned above by making a C- shape annuloplasty ring actually a D-shape annuloplasty ring with the connection of the C- shaped annuloplasty ring ends with the link 51. It is obvious that the scope of the invention remains the same since the principle that the basic functional units 38,39,40,41,42,43,44 are connected to each other with non-conductive material 45,46,47,48,49,50 is applied. The link's 51 main advantage is that doesn't apply pressure on the heart's conduction system or the aortic valve because it is made out of flexible and non-rigid material. This is better shown in Figs 19,20,21 where the insufficiency of the tricuspid valve 91 due to it's annulus stretch 92 is being corrected with a D-shape annuloplasty ring 94 leading to a restored valve 93 and the insufficiency of the mitral valve 82 due to it's annulus stretch 83 is being corrected with a closed shape annuloplasty ring (circular prosthesis) 85 similar to the one shown in Fig.5 leading also to restored mitral valve 84 as shown in Figs 15,16,17.
In this point it worth mentioning that the formations (circular prosthesis, D-shape prosthesis) of the annuloplasty rings that are described above and shown in the Figs 5,9 are only for explanatory reasons and may vary depending on the application.

Additionally, it is noteworthy, that the mitral valve's annulus naturally conforms to a saddle shape in systole. This configuration is believed to put the leaflets into a lower-energy equilibrium with the annulus and sub-valvular apparatus. Conventional flat annuloplasty rings restrict posterior leaflet motion, which may result in a "monocusp" valve, affecting valvular stress distribution. It is hypothesized that saddle-shaped annuloplasty rings cause less distortion of the physiologic leaflet geometry than do flat rings. In this point is worth mentioning that our annuloplasty device since is comprised out of non rigid compartments while is being sewed on the valve's annulus can resemble the annulus structure and conform to a saddle shape .Due to nitinol's unique characteristics (pseudo-elasticity, flexibility), when the annuloplasty ring described in this patent, is being sewed on the mitral's valve annulus can follow it's saddle shape structure leading to better restoration.

In order to be easier for someone to understand the way that the annuloplasty ring described in this patent works, below follow two indicative examples of it's application on both the mitral and the tricuspid valve.

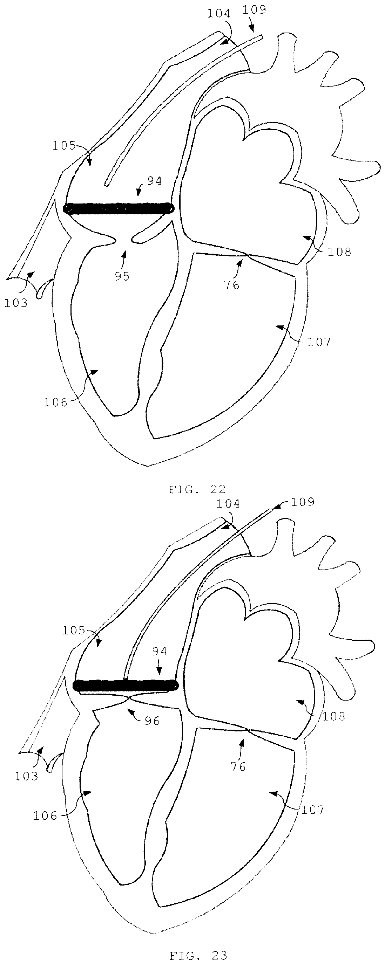

After confirming the valve's 91 insufficiency (which is followed by blood regurgitation from the right ventricle 106 to the right atrium 105 during systole) and all the other necessary parameters needed for a surgery of that kind to be performed are covered, the implantation of the annuloplasty ring 94 around the tricuspid valve's annulus 92 is about to take place. During surgery a D-shape annuloplasty ring 94 of specific size is being selected and sewed around the valve's annulus 92, reducing it's perimeter thus correcting the insufficiency leading to repaired tricuspid valve 93 as shown in Fig 19. However, as mentioned before, complications such as persisting regurgitation or local stenosis are not rare after the valve's annulus total circumference reduction and actually are common side effects caused by conventional annuloplasty rings. The annuloplasty ring described in this patent though, since it's circumference can be partially reduced in vivo during surgery or after the end of it (follow-up) doesn't cause these kind of complications and is actually leading to better kinematics between the valvular leaflets, thus improving the current surgical technique. So as far as the tricuspid valve is concerned, activation of the annuloplasty ring that is sewed on it's annulus is carried out the way described below. First, a catheter 109 of certain design is being inserted to the right atrium 105, as shown in Fig 22, via the superior 104 or inferior 103 vena cava and it's properly designed head meets the upper side of a certain catheter's socket. In this point is worth mentioning that the catheter can be inserted to the right atrium in any possible way (transdermal, transseptal etc.) and the one described here (via the superior vena cava) is just for explanatory reasons. During that procedure due to each catheter's socket contrast outer layer the invasive cardiologist, depending on the kind of correction it needs to be performed, can easily guide the catheter and eventually connect it with the suitable catheter's socket. Furthermore due to the catheter's socket magnetic base the process described above is facilitated. Then, after the catheter's socket and the catheter's connection as shown in Fig 23, the transportation of electric energy from an outer source to the corresponding functional unit can begin. Due to the heating wire's properties, the temperature is locally risen at a high speed and is inductively transmitted to the corresponding spring made out of nitinol. As a result the nitinol spring will start shrinking in size until it returns to it's initial position causing the local reduction of the valve's annulus circumference, thus correcting the valve.

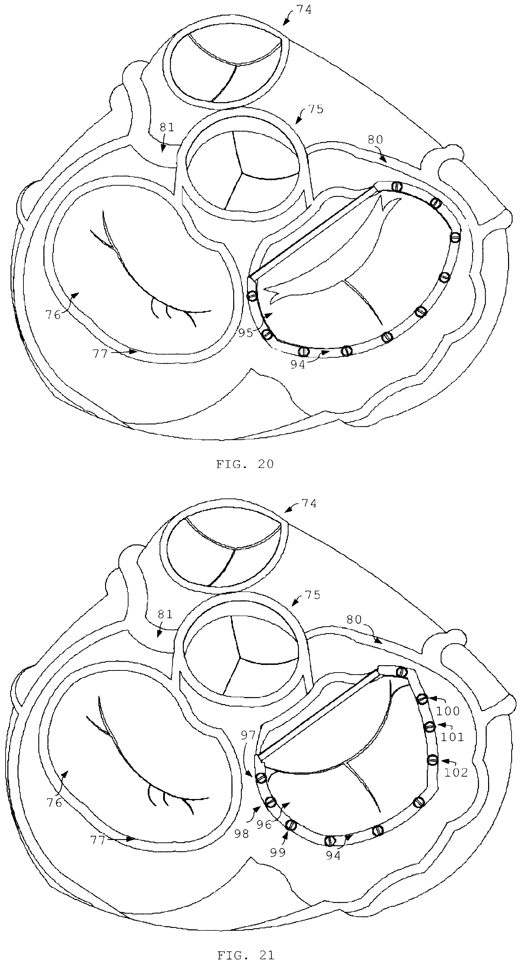

Although the initial regurgitation is corrected, the repaired tricuspid valve 93 might suffer a new insufficiency 95 as shown in Fig 20. The great advantage of the annuloplasty ring described in this patent is that the invasive cardiologist can re-adjust the circumference of the annuloplasty ring in vivo, years after the initial implantation, thus correcting the new insufficiency the same way described above .To be more specific the basic functional units 97,98,99 and 100,101,102 of the annuloplasty ring 94 are activated thus shrinking in size, re-adjusting the annulus shape in a way that no insufficiency remains 96 as shown in Fig 21.

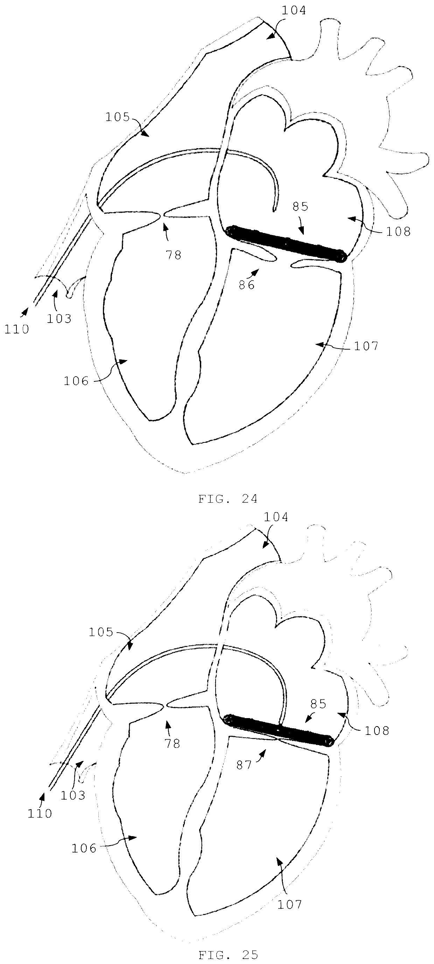

As it is obvious, the same thing applies to the mitral valve, where now a circular prothesis is chosen. After confirming the valve's insufficiency 82 (which is followed by blood regurgitation from the left ventricle 107 to the left atrium 108 during systole)as shown in Fig 14 and all the other necessary parameters needed for a surgery of that kind to be performed are covered, the implantation of the annuloplasty ring 85 around the mitral valve's annulus 83 is about to take place. During surgery a closed shape annuloplasty ring 85 of specific size is being selected and sewed around the valve's annulus 83, reducing it's perimeter thus correcting the insufficiency. However, as mentioned before, complications such as persisting regurgitation or local stenosis are not rare after the valve's annulus total circumference reduction and actually are common side effects caused by conventional annuloplasty rings. The annuloplasty ring described in this patent though, since it's circumference can be partially reduced in vivo during surgery or after the end of it (follow-up) doesn't cause these kind of complications and is actually leading to better kinematics between the valvular leaflets, thus improving the current surgical technique. So as far as the mitral valve is concerned, activation of the annuloplasty ring that is sewed on it's annulus is carried out the way described below. First, a catheter 110 of certain design is being inserted transseptaly to the left atrium 108 as shown in Fig 24 and it's properly designed head meets the upper side of a certain catheter's socket. In this point is worth mentioning that the catheter can be inserted to the left atrium in any possible way (transdermal, transseptal etc.) and the one described here (through the septum) is just for explanatory reasons. During that procedure due to each catheter's socket contrast outer layer the invasive cardiologist, depending on the kind of correction it needs to be performed, can easily guide the catheter and eventually connect it with the suitable catheter's socket. Furthermore due to the catheter's socket magnetic base the process described above is facilitated. Then, after the catheter's socket and the catheter's connection as shown in Fig 25, the transportation of electric energy from an outer source to the corresponding functional unit can begin. Due to the heating wire's properties, the temperature is locally risen at a high speed and is inductively transmitted to the corresponding spring made out of nitinol. As a result the nitinol spring will start shrinking in size until it returns to its initial position causing the local reduction of the valve's annulus circumference, thus correcting the valve.

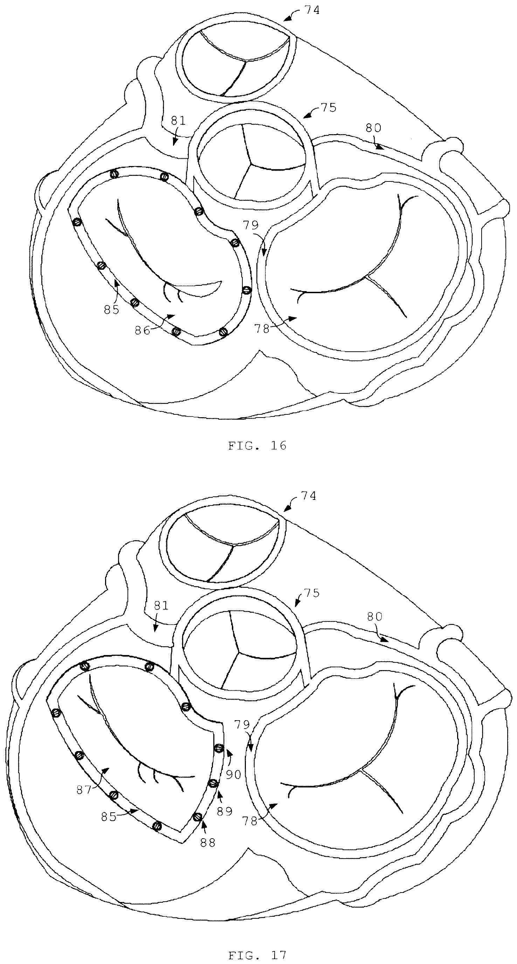

Although the initial regurgitation is corrected, the repaired mitral valve 84 might suffer a new insufficiency 86 as shown in Fig 16. The great advantage of the annuloplasty ring described in this patent is that the invasive cardiologist can re-adjust the circumference of the annuloplasty ring in vivo, years after the initial implantation, thus correcting the new insufficiency the same way described above . To be more specific the basic functional units 88,89,90 are activated, thus shrinking in size re-adjusting the annulus shape in a way that no insufficiency remains 87 as shown in Fig 17.

Although the invention has been explained to it's preferred embodiments as mentioned above, it is to be understood that many other possible modifications and variations can be made without departing from the scope of the present invention. It is, therefore, contemplated that the appended claim or claims will cover such modifications and variations that fall within the true scope of the invention.

## Claims

1. A selectively adjustable annuloplasty ring, comprising:
A sewing cuff 17 that surrounds a plurality of basic functional units 18-27 connected to each other with non-conductive material 28-37 and have the ability to shrink autonomously in size when energy is applied via catheterization causing the ring's partial circumference reduction thus correcting blood regurgitation.

2. The selectively adjustable annuloplasty ring in accordance with claim 1 wherein:
Each basic functional unit 1 consists of a nitinol spring 2,a heating wire 3 mounted on the nitinol spring 2 intended to transfer heat inductively to the nitinol spring 2 and a catheter's socket 4.

3. The selectively adjustable annuloplasty ring in accordance with claim 2 wherein:
The energy delivery circuit consists of a heating wire 3 mounted on the inside of the nitinol spring 2 and a catheter's socket 4 mounted on the outer surface of the sewing cuff 17.

4. The selectively adjustable annuloplasty ring in accordance with claim 2 wherein:
The heating wire 3 has the form of a smaller spring inside the nitinol spring and it's ends after being coiled around the ends of the nitinol spring are passing through the sewing cuff 17 and are being connected to the catheter's socket 4 magnetic base 8,9.

5. The selectively adjustable annuloplasty ring in accordance with claim 2 wherein:
The heating wire 3 is constructed out of materials like Kanthal, Nichrome, Constantan ,Balco etc that have the ability to rise their temperature really fast without energy loss.

6. The selectively adjustable annuloplasty ring in accordance with claim 2 wherein:
The catheter's socket bottom is made out of magnetic bodes 8,9 that provide stability between the catheter and the catheter's socket 4.

7. The selectively adjustable annuloplasty ring in accordance with claim 2 wherein:
The catheter's socket 4 majority is constructed out of insulating material 5 thus enabling the energy transportation only between the catheter and the catheter's socket magnetic base 8,9 , minimizing the complications.

8. The selectively adjustable annuloplasty ring in accordance with claim 2 wherein:
The catheter's socket 4 outer layer is covered by radiopaque marker 6 thus enabling the distinction between them during catheterization.

9. The selectively adjustable annuloplasty ring in accordance with claim 1 wherein:
The inner layer 52 of the sewing cuff 17 is made out of heat insulating material thus avoiding tissue damage due to energy leak during activation of the ring while the outer layer 53 is made out of double knitted velour polyester.

10. The selectively adjustable annuloplasty ring in accordance with claim 1 wherein:
It can take only closed formations since only closed shapes can reduce their diameter by reducing their circumference.

11. The selectively adjustable annuloplasty ring in accordance with claim 1 wherein:
It can take the shape of any cardiac valve's annulus that is sewed on due to the properties (flexibility-elasticity) of the materials composing it and can be fully closed or of D shape without applying any kind of pressure to the surrounding tissues, due to it's specific construction, as described above.

12. The selectively adjustable annuloplasty ring in accordance with claim 1 wherein:
The energy applied by the catheter affects only the basic functional unit on whose catheter's socket the catheter is connected because of the insulating material interjected between the heat sensitive nitinol springs thus providing total and selective control of the ring's circumference adjustment.

13. The selectively adjustable annuloplasty ring in accordance with claim 1 wherein:
The length of any of the basic functional units is decreased in a linear way, when activated, meaning that the nitinol spring decreases it's length as long as heat is transmitted to it and can be stopped to any one of the intermediate positions.

14. The selectively adjustable annuloplasty ring in accordance with claim 1 wherein:
The re -adjustment of the annuloplasty's ring circumference is conducted in vivo via catheterization without an open heart procedure.
